# EUROPEAN PATENT APPLICATION

(11) **EP 1 864 658 A1**
(43) Date of publication of application: **12.12.2007**
(21) Application number: 06730917.9
(22) Date of filing: 31.03.2006
(51) Int. Cl.: A61K 31/355, A61K 31/122, A61P 9/00, A61P 43/00

(54) **AGENT FOR ALLEVIATING VASCULAR INSUFFICIENCY**

(30) Priority: 31.03.2005 JP 2005104293
(71) Applicant: FUJI CHEMICAL INDUSTRY CO., LTD., Nakaniikawa-gun, Toyama 930-0397 (JP); Higashi, Naoki, 1-47-18, Den-en chofu, Ota-ku Tokyo 9300397 (JP)
(72) Inventor: TAKAHASHI, Jiro, Nakaniikawa-gun Toyama 9300397 (JP); HIGASHI, Naoki, Tokyo 145-0071 (JP)
(74) Representative: Albrecht, Thomas
(86) International application number: PCT/JP2006/306969
(87) International publication number: WO 2006/106986

(57) **Abstract**

[Object] In recent years, life style-related diseases, particularly circulatory system diseases caused by the tendency toward high caloric diet, the change in life style, lack of exercise, stress and so on increased and have became a serious social problem. These diseases are caused mainly by injures in blood vessels. To prevent injures in blood vessels, there has been required a vascular endothelial cell-protecting agent and a food having an effect of protecting vascular endothelial cells.

[Means to solve] It is possible to provide an agent for alleviating, treating and preventing vascular failure and a vascular endothelial cell-protecting agent **characterized by** containing, as an effective ingredient, at least one active oxygen scavenger such as astaxanthin and tocotrienols; and a food, a drink and an animal feed each having an effect of alleviating, treating and preventing vascular failure and an effect of protecting vascular endothelial cells **characterized by** containing, as the effective ingredient, at least one active oxygen scavenger such as astaxanthin and tocotrienols.

## Description

### Technical Field

The present invention relates to an agent for alleviating, treating and preventing vascular failure and a vascular endothelial cell-protecting agent which are characterized by containing at least one of astaxanthin and tocotrienols as an effective ingredient, relates to a food and a drink having an effect of alleviating, treating and preventing vascular failure and an effect of protecting vascular endothelial cells which are characterized by containing at least one of astaxanthin and tocotrienols as an effective ingredient, and relates to an animal feed having an effect of alleviating, treating and preventing vascular failure and an effect of protecting vascular endothelial cells which are characterized by containing at least one of astaxanthin and tocotrienols as an effective ingredient.

### Background Art

In recent years, increases in life style-related diseases caused by the tendency toward high caloric diet, the change in life style, lack of exercise, stress, etc. became a serious social problem. Especially, heart disease, derebrocascular accident, kidney failure, diabetes and so on which occur as the result of development of life style-related diseases occupy more than 30 percent of the cause of death and there is a tendency to increase the percentage of the cause of death from now. Even though they do not become the cause of death, a variety of diseases such as metabolic syndrome, hyperpiesia, cardiovascular, hyperlipemia, arteriosclerosis, diabetes, etc. bring about complications such as nephropathy, eyesight injury, nerve injury, lowering of resistance, etc. and lower the quality of life greatly. These diseases are caused by injures in vascular failure. Specifically, the cause is that attack substances such as peroxide lipid, active oxygen and so on which are present in the body injure not only vascular endothelial cells and but also the inside of vascular tissue. An inducer of the initial stage was proved to be oxidative stress of cell. Also, not only a vascular endothelial cell protects merely vascular tissue by covering the inside of vascular lumen but also it is endocrine organ covering the whole body and involving in the function of transporting a material from vascular lumen to subendothelium and in function or factor which is linked directly with life-support such as blood pressure regulation, bloodstream regulation, blood coagulation, etc. and hence its protection is important.

In recent year, astaxanthin which is a kind of the same carotenoid with β-carotene and a red pigment distributed widely in natural origin especially in ocean as in crustaceans such as shrimp, crab, etc.; fishes such as a salmon, a porgy, etc.; algae such as green alga of Haematococcus, etc.; yeasts such as *Phaffia* red yeast, etc. and which has a rich experience as food has been found to have antioxidative action that is about 1000 times as strong as vitamin E and about 40 times as strong as β-carotene. At the present time, astaxanthin goes so far as to be expected as healthy food by the business world unlike the time hitherto dealt with merely as pigment.

As other functional characteristics which astaxanthin has, there have been made various reports such as its antiinflammatory action, anti-arteriosclerotic action, mneme-enhancing action, diurnal rhythm adjusting action, immunopotentiation action, anti-stress action, action of increasing duration of muscle function, action of protecting retina from photic injury, action of improving accommodation, sperm's quality improving action, etc.

Tocotrienol occurs in wheat plants rice bran palm oil and so on, and is a compound which closely resemble tocopherol in the structure wherein three double bonds are entered into the side chain part of tocopherol. This compound is a natural material having a rich experience as food like the above-mentioned astaxanthin, too. With respect to the physiological activity of tocotrienol, antioxidative action is taken like astaxanthin. The action is said to be about 50 times as strong as tocopherol.

As other function of tocotrienol, its cholesterol-lowering action, action of inhibiting cell of breast cancer from propagating and so on have been reported. Very recently, there were discovered new functional properties such as action of inhibiting neovasculaization, action of improving of general blood flow, action of improving defomation ability of eryyhrocytic membranes and so on which tocopherol does not have. Also, it is widely used in Europe and America as vitamin E of the next generation for external use such as cosmetics and the like. This tocotrienol may be obtained by methods to squeeze the natural material, to be extracted from the natural material, by a synthesis method and so on. In general, it may be extracted from the rind and/or seed of Palmae. Tocotrienol obtained from the natural material by extraction is a mixture of plural isomers thereof. By utilizing its antioxidant action the application of it to food additive, cosmetics and so on has been conducted.

It is known that a drink and a food to which astaxanthin has added suppress the oxidation of low-density lipoprotein (LDL) which is present in serum thereby exerting effect of suppressing arteriosclerosis, ischemic heart diseases or ischemic encephalopathy (Patent literature 1). It is known that astaxanthin has effects of suppressing the oxidative damage of erythrocytes, preventing the hardening of erythrocytes and stabilizing erythrocytes (Patent literature 2).

However, it has not yet been known that by administering at least one of astaxanthin and tocotrienols a vascular endothelial cell is protected and vascular failure is alleviated, treated and prevented.
Patent literature 1: JP10-155459 A
Patent literature 2: JP2002-226368 A

### Disclosure of the invention

### Subject Matter to be Solved by the Invention

As a result of having searched for a substance to alleviate vascular failure and to protect a vascular endothelial cell in order to solve the above-described object, the present inventors have found that astaxanthin and tocotrienols have effects of alleviating vascular failure and protecting a vascular endothelial cell. The present invention has been completed based on such finding, and it provides an agent for protecting a vascular endothelial cell and an agent for alleviating vascular failure whose effective ingredient are astaxanthin and tocotrienols and it further provides a drink, a food and an animal feed, each having effects of protecting a vascular endothelial cell and alleviating vascular failure which contain astaxanthin and tocotrienol as an effective ingredient.

An object of the present invention is to provide an agent for alleviating vascular failure and an agent for protecting a vascular endothelial cell which are characterized by each containing at least one of astaxanthin and tocotrienols as an effective ingredient and an another object of the present invention is to provide a drink, a food and an animal feed, each having effects of alleviating vascular failure and protecting a vascular endothelial cell, each containing astaxanthin and tocotrienols as an effective ingredient. The medicament, drink, food and animal feed of the present invention alleviate vascular failure and protect a vascular endothelial cell whereby they are useful in alleviating, treating, suppressing and preventing diseases caused by the vascular failure and by injury or rupture of the vascular endothelial cell.

### Means for Solving the Subject Matter

As a result of having ardently studied to solve the above-described objects, the present inventors have found that when at least one of astaxanthin and tocotrienols is administered, each of them shows effects of alleviating vascular failure and of protecting a vascular endothelial cell. The present invention is based on such finding.

That is, the present invention is:
(1) an agent for alleviating vascular failure which is characterizing by containing at least one of astaxanthin and tocotrienols as an effective ingredient,
(2) a vascular endothelial cell-protecting agent which is characterizing by containing at least one of astaxanthin and tocotrienols as an effective ingredient,
(3) a drink and a food each having an effect of alleviating vascular failure which is characterizing by each containing at least one of astaxanthin and tocotrienols as an effective ingredient,
(4) a drink and a food each having an effect of protecting a vascular endothelial cell which is characterizing by each containing at least one of astaxanthin and tocotrienols as an effective ingredient,
(5) an animal feed having an effect of alleviating vascular failure which is characterizing by containing at least one of astaxanthin and tocotrienols as an effective ingredient, and
(6) an animal feed having an effect of protecting a vascular endothelial cell which is characterizing by containing at least one of astaxanthin and tocotrienols as an effective ingredient.

### Effect of the Invention

The term "astaxanthin" in the present invention is meant one derived from natural origin and one obtained by synthesis. Examples of one derived from natural origin include derived from one from crusts, eggs and organs of crustaceans such as shrimp, krill, crab and the like; skins and eggs of various fishes and shellfishes; algae such as green alga of Haematococcus, etc.; yeasts such as *Phaffia* red yeast, etc.; oceanic bacteria; and seed plants such as *Adonis* amurensis and *Ranunculus acris.* An extract from natural origin and a chemically synthesized product are put on the marketplace and hence they are easily available.

Astaxanthin can be obtained by cultivation of e.g. *Phaffia* red yeast, Hematococcus alga, oceanic bacteria, etc. in an appropriate medium in accordance with the known method. Green alga of Hematococcus is the most preferred from the viewpoints of easiness of cultivation and extraction, astaxanthin contained at the highest concentration and high productivity.
As the cultivation method for obtaining Hematococcus algae having high astaxanthin content, a method cultivating Hematococcus algae using hermetic type of cultivation apparatus is preferable because there is no possibility for different kind of microorganisms to be mixed therein and propagate and because the possibility of other contaminants to be mixed therein is little. For example, a cultivation process using a partially openable type of dome shaped, conical or cylindrical cultivation apparatus wherein culture incubators are equipped with an optionally movable gas ejector (WO 99/50384), a cultivation process wherein a light source is placed in a hermetic type of cultivation tank and cultivation is conducted under radiation of light from the inner part of a cultivation tank, and a cultivation process using plate-like cultivation tank are suitable.

Various processes are known for extracting astaxanthin from the before-described cultured material or the before-described crustaceans and for purifying the extract. Since astaxanthin and ester thereof are oil soluble substances, astaxanthin containing component may be extracted with an oil soluble organic solvent such as acetone, alcohol, ethyl acetate, benzene, chloroform or the like. Also, a superclinical extraction may be conducted with carbon dioxide or water. After extraction, a solvent is removed according to the conventional manner thereby there can be obtained a mixed and concentrate of astaxanthin of monoester type and astaxanthin of diester type. The obtained concentrate may be further purified by separation column or decomposition with lipase, if desired.

The extraction process of astaxanthin as described below is preferred because of contaminants being small and because of the high contents of astaxanthin and triglyceride each having good purity. The Hematococcus algae cultured using the before-described dome type of cultivation apparatus or hermetic type of cultivation tank is dried and pulverized and thereafter is subjected to extraction with acetone or otherwise the pulverization and extraction are conducted in acetone at the same time, and acetone is removed.

As the form to use astaxanthin, the astaxanthin extracts obtained by the before-described processes, powder or aqueous solution each containing them, or dried products of green alga of Haematococcus, of *Phaffia* red yeast, of oceanic bacteria, etc. and pulverized products thereof may be used.

Astaxanthin is 3,3'-dihydroxy-β, β-carotene-4, 4'-dione and has stereoisomers. Specifically, such three stereoisomers are known as (3R, 3'R)-astaxanthin, (3R, 3'S)-astaxanthin and (3S, 3'S)-astaxanthin. Any of them can be used in the present invention.

Unless otherwise described herein, astaxanthin includes astaxanthin and/or its ester. Furthermore, ester of astaxanthin includes monoester and/or diester.

It is known that astaxanthin has not been observed having any mutagenicity and is highly safe compound and it has been widely used as food additive (Jiro Takahashi et al; toxicity test of Hematococcus alga astaxanthin- Ames test, rat single dose toxicity test, rat 90-days repeat dose oral toxicity test- Journal of Clinical Therapeutic and Medicine, 20:867-881, 2004).

For the medicament of the present invention wherein astaxanthin is an effective ingredient, there can be used at least one of free form, monoester form and diester form of astaxanthin.
The diester form is physically more stable than the free or monoester form and hard to be subjected to oxidative decomposition in an animal feed, because its two hydroxy groups are protected by ester bondage. However, when it is taken into the living body, it is considered quickly hydrolyzed into free astaxanthin by bioenzymes to exert its effect.

As a monoester of astaxanthin, there can be taken monoesters esterified with lower or higher saturated fatty acid, or lower or higher unsaturated fatty acid. Specific examples of lower or higher saturated fatty acid, or lower or higher unsaturated fatty acid include acetic acid, lauric acid, myristic acid, pentadecanoic acid, palmitic acid, palmitoleic acid, heptadecanoic acid, elaidic acid, ricinoleic acid, petroselinic acid, vaccenic acid, eleostearic acid, punicinic acid, licanoic acid, palynalic acid, gadolic acid, 5-eicosenoic acid, 5-docosenoic acid, cetolic acid, ercinoic acid, 5,13-docosadienoic acid, selacholic acid, decenoic acid, stering acid, dodecenoic acid, oleic acid, stearic acid, eicosapentaenoic acid, docosahexaenoic acid, linoleic acid, linolenic acid, arachidonic acid, etc.
As a diester of astaxanthin, there can be taken diesters esterified with the same or different fatty acids selected from the above fatty acids.

Furthermore, as monoester of astaxanthin, there can be taken monoesters esterified with an amino acid such as glycine, alanine or the like; with a mono- or poly-carboxylic acid such as acetic acid, citric acid or the like; with an inorganic acid such as phosphoric acid, sulfuric acid or the like; with a saccharide such as glucoside or the like; with a glyco-fatty acid such as glycoglycero-fatty acid or sphingoglyco-fatty acid; with a fatty acid such as glycero-fatty acid, with glycero-phosphoric acid, etc. In case where it is to be considered, salt of the above monoester is included.

As a diester of astaxanthin, there can be taken diesters esterified with the same or different acids selected from the above lower saturated fatty acid, higher saturated fatty acid, lower unsaturated fatty acid, higher unsaturated fatty acid, an amino acid, a mono- or poly-carboxylic acid, an inorganic acid, a sugar, a glyco-fatty acid, fatty acid and glycero-phosphoric acid. In case where it is to be considered, salt of the above diester is included. As a diester of glycero-phosphoric acid, there may be taken saturated fatty acid ester of glycero-phosphoric acid or esters of glycero-phosphoric acid containing fatty acid selected from higher unsaturated fatty acid, unsaturated fatty acid and saturated fatty acid.

The term "tocotorienols" in the present invention includes isomers or derivatives of tocotorienol and isomers or derivatives of tocopherol, and is meant one derived from natural origin and one obtained by synthesis.

Tocotorienol means α-tocotrienol, β-tocotrienol, γ-tocotrienol, δ-tocotrienol, isomers thereof and their esters with niconitic acid, acetic acid, succinic acid and the like. These tocotorienols include d-, 1- or dl- type of isomers. They may be also used in mixture of two or more kinds.

These tocotorienols may be obtained by the conventional method, for example, one squeezing natural material, extraction from the natural material, synthesis process or the like. These tocotorienols may be further subjected to separation and purification to make the purity better by column chromatography, if desired.

As tocopherol, there are tocopherol and its derivative, and an oil containing at least one of them. It means α-tocopherol, β-tocopherol, γ-tocopherol, δ-tocopherol, isomers thereof and their esters with niconitic acid, acetic acid, succinic acid and the like. They may be also used in mixture of two or more kinds.

The term "vascular failure" in the present invention is a generic name for failure in vascular endothelial function, including failure in vascular smooth muscle function, vascular metabolic failure and the like. As specific injury, vasospasm by endothelium anaclitic smooth muscle laxity injury, monocyte adhesions by increase of adhesive molecule expression, formation of thrombus by lowering of fibrinolytic system are taken.

The followings illustrate the present invention specifically.
The term "alleviation of vascular failure" in the present invention is an effect of alleviating, treating and preventing injuries in various vascular functions caused by a variety of factors. For example, it includes an effect of alleviating, treating and preventing injures in such functions of vascular endothelial cell as in the function adjusting bloodstream by perceiving signal or stress from organs, in the function repairing blood vessel when injured, in the function of various incretions, in the function transporting various substances between vascular lumen and subendothelialium, in the function protecting subendothelial tissue from attack substances present in blood. More specifically, it include an effect of alleviating, treating and preventing injures in the vascular subendothelial function such as vasospasm by endothelium anaclitic unstriated muscle laxity injury, monocyte adhesion by development and increase of adhesive molecule, formation of thrombus by lowering of adenoid system. The terms "protection of a vascular endothelial cell" is an effect to protect a vascular endothelial cell from the attack substances such as peroxide lipoid, active oxygen and the like, and from physical attack such as high blood pressure, etc.

The agent for alleviating vascular failure and the protecting a vascular endothelial cell-protecting agent in the present invention may be administered orally or parenterally. As oral dosing preparations, they may be administered in solid dosing forms such as tablet, intraoral disinteegratale tablet, capsule, granule, fine granule and the like and in liquid dosing forms such as syrup and suspension and the like. As parenteral dosing preparations, they may be administered in the forms of parenteral, eye drops, nose drops, patch, paste and suppository. The lipid-dispersed type of preparation is effective for increasing the concentration in blood.

The agent for alleviating vascular failure and the vascular endothelial cell-protecting agent in the present invention may contain the appropriate amount of various additives which are used for production of general preparation. Examples of such additives include an excipient, a binding agent, an acidifier, an effervescent agent, an artificial sweetener, a perfume, a lubricant, a coloring agent, a stabilizer, a pH adjusting agent, a surfactant and so on. Examples of an excipient include starches such as corn starch, potato starch, wheat starch, rice starch, partially pregelatinized starch, pregelatinized starch, porous starch and the like; saccharides such as lactose, sucrose, glucose and the like; sugar alcohols such as mannitol, xylitol, erythritol, sorbitol, maltitol and the like; inorganic compounds such as magnesium aluminometasilicate, hydrotalcite, calcium phosphoric acid anhydride, precipitated calcium carbonate, calcium silicate, light anhydrous silicic acid and the like. Examples of a binding agent include hydroxypropyl cellulose, hydroxypropylmethyl cellulose, polyvinylpyrrolidone, gum arabic, gelatin, pullulan, etc. Examples of a disintegrant include starch, agar, starch, methylcellulose, carmellose calcium, sodium carboxymethyl starch, cross carmellose sodium, crospovidone, crystalline cellulose, etc. Examples of an acidifier include citric acid, tartaric acid, malic acid, ascorbic acid and the like. Examples of an effervescent agent include sodium hydrogencarbonate, sodium carbonate and the like. Examples of a sweetener include saccharine sodium, dipotassium glycyrrhizinate, aspartame, stevia, thaumatin, etc. Examples of a perfume include lemon oil, orange oil, menthol, etc. Examples of a lubricant include magnesium stearate, sucrose fatty acid ester, polyethylene glycol, talc, stearic acid, sodium stearyl phthalate, etc. Examples of a coloring agent include food pigments such as food yellow No. 5, food red No. 2, food blue No.2 and the like; food lake pigment, ferric oxide, etc. Examples of a stabilizer include disodium edatate, tocopherol, cyclodextrin, etc. Example of a pH adjusting agent include citrate, phosphate, carbonate, tartrate, fumarate, acetate, salt of amino acid, etc. Examples of a surfactant include polysorbate 80, methyl cellulose, hydroxyethyl cellulose, sodium carboxymethyl cellulose, polyoxyethylene-sorbitan monolaurate, gum arabic, powdered traganth and the like. It is preferable to incorporate them in powdered state for making the absorbability of astaxanthin or tocotrienol good and making preparation easy.

Liquid dosing preparations such as syrup, drink, suspension, eye drop, injection and the like may be prepared by the conventional manner compounding the effective ingredient in the presence of a pH adjusting agent, buffering agent, solubilizer, suspension, isotonizing agent, stabilizer, antiseptic depending on necessity. Examples of a suspension include polysorbate 80, methyl cellulose, hydroxyethyl cellulose, sodium carboxymethyl cellulose, polyoxyethylenesorbitan monolaurate, gum arabic, powdered traganth and the like. Examples of a solubilizer include polysorbate 80, polyoxyethylene hydrogenated caster oil, nicotinic acid amide, polyoxyethylenesorbitan monolaurate, macrogol, caster oil fatty acid ethyl ester and the like. Examples of a stabilizer include sodium sulfite, sodium metasulfite and the like. Examples of an antiseptic include methyl p-hydroxybenzoate, ethyl p-hydroxybenzoate, sorbic acid, phenol, cresol, chlorocresol and the like.

In order to enhance effects of alleviating vascular failure and protecting a vascular endothelial cell in the present invention, there may be added a compound having antioxidative action. An antioxidant agent is considered to enhance the effect of astaxanthin or totrienols by suppressing the oxidation of astaxanthin or totrienols in the medicament of the present invention and by suppressing the oxidation of astaxanthin or totrienols in the living body. Antioxidant is not particularly limited. Any one can be applied if it has antioxidative action. There can be selected at least one of antioxidants from the group consisting of vitamin A substances such as gluthatione, retinol, 3,4-dihydroxyretinol and the like; vitamin B; vitamin C substances such as D-ascorbic acid, L-ascorbic acid and the like; Vitamin E substances such tocopherol, tocotrienol, vitamin E acetate, vitamin E succinate; vitamin E phosphates; carotenoids such as β-carotene, rutin and the like, and pharmaceutically acceptable salts thereof; coenzyme, flavonoid, tannin, ellagic acid, polyphenols, nucleic acids, herb medicines, marine algae, inorganic substances, and mixture thereof. Gluthatione is preferred. Also, similar effect can be obtained by incorporating a fruit or algae or bacteria each containing the above antioxidant. The amount incorporated of the compound having antioxidative action is 0.01-1,000 times, preferably 0.1-100 times based on the total amount of astaxanthin and/or tocotrienol.

Also, for a skin external application agent, in addition to the above-described ingredients, there may be adequately incorporated ingredients which are usually used for skin external application agent depending on the necessity. Example of such ingredients include a whitening agent, a humectant, an antioxidant, an oil component, an ultraviolet absorber, a surfactant, a viscosity-increasing agent, alcohols, a powder component, a coloring agent, an aqueous component, water, various skin nutrients, etc.

The amount of astaxanthin for use as an agent for alleviating vascular failure and a vascular endothelial cell-protecting agent is 0.5-100mg, preferably 1-20 mg in terms of free astaxanthin per day for adult, and an oral or parenteral administration is conducted in such dose. The dosage may vary in age, body weight or grade of symptoms of a patient to be administered and the dosing form. The astaxanthin content in the medicament of the present invention may be 0.01-99.9 % by weight, preferably 0.1-90 % by weight.

Although the amount of tocotrienols for use as an agent for alleviating vascular failure and a vascular endothelial cell-protecting agent is varied by ocotrienol, tocopherol and derivatives thereof, it is 0.5-300 mg, preferably 1-20 mg per day for adult, and an oral or parenteral administration is conducted in such dose. The dosage may vary in age, body weight or grade of symptoms of a patient to be administered and the dosing form. The tocotrienol content in the medicament of the present invention may be 0.01-99.9 % by weight, preferably 0.1-90 % by weight.

In the case where both of astaxanthin and tocotrienols are incorporated in an agent for alleviating vascular failure and a vascular endothelial cell-protecting agent, they may be incorporated in a ratio of 0.1-20 parts by weight, preferably 0.5-10 parts by weight of tocotrienols to 1 part by weight of astaxanthin. The total content of astaxanthin and tocotrienol in the medicament of the present invention may be 0.01-99.9 % by weight, preferably 0.1-90 % by weight.

Since the agent for alleviating vascular failure and a vascular endothelial cell-protecting agent are capable of protecting blood vessels and adjusting blood vessels-producing factor, they have an effect of treating, improving and preventing diseases which are said to be caused by the abnormality in blood vessels and its related factor. Examples of such diseases include pulmonary emphysema, gastric ulcer, gastritis, hepatitis, pancreatitis, nephritis, other inflammatory diseases, cataract, Alzheimer's disease, aging, hidrosis, ischemic disease, complications of diabetes being nerve injury and retinopathy, kidney disease, great vessel injury, and blood disease. In nerve injury, they are effective in treating improving and preventing sudden bradyacusia, abnormality of eye and face (paralysis and pain), orthostatic hypotension, abnormality of perspiration, flux and obstipation (digestive trouble), dysuria, pain of membrum, pareatrophy, paresthesia, atrophy of muscle, ED, asthenopia, muscle fatigue, contraction of muscle and melancholia. In retinopathy, they are effective in treating improving and preventing macular degeneration, glaucoma, cataract, simple retinosis, preproliferative retinopathy, and proliferative retinopathy. In immune diseases, they are effective in treating improving and preventing autoimmune diseases (allergic disease, vermination, systemic lupus erythematosus, rheumatoid arthritis, Behcet's disease), virus or bacteria infection, malignant tumor (plasmacytoma, multiple myeloma, cancer dyscrasia, atrial myxoma, mycloma, Lennert's lymphoma, etc.), HVC disease or acquired immune deficiency, Kaposi's sarcoma, postclimacteric osteoporosis, inflammatory skin disease (hyperkeratosis, atopic dermatitis, contact dermatitis, etc.), inflammatory bowel diseases (ulcerative colitis, etc.), inflammatory liver diseases (hepatitis B, hepatitis C, alcoholic heptatitis, etc.), inflammatory kidney diseases (glomerular nephritis etc.) and inflammatory respiratory diseases (asthma, chronic obstructive lung disease, bronchitis, etc.).

As to other uses of the agent for alleviating vascular failure and a vascular endothelial cell-protecting agent in the present invention, they may be used as organ-preserving and protecting agents owing to their effect of protecting vascular cell. An organ to be used by the organ preserving and protecting agents is all ones of a human and an animal. Examples of such organ include heart, kidney, pancreas, lung, lever of a human and an animal. In preserving the organ ablated from a donor during the organ transplantation, they may be used as the additive to a preservation medium or a perfusion in order to prevent injury in the organ to the minimum. Also, they may be administered to a patient to be transplanted as the organ-protecting agent for suppressing or preventing a rejection of the posttransplant. Furthermore, by using the preserving agent of the present invention, the ablated organ can be preserved without being subjected to deterioration and the function of the organ can be kept up to after transplantation. Incidentally, in case where the ablated organ is preserved as the purpose of transplantation, the preserving agent of the present invention is administered to a donor in advance as it is effective to keep the vascular cell healthy and normal.

The present invention includes a food and a drink each having an effect of alleviating, treating and preventing vascular failure and an effect of protecting vascular endothelial cells which are characterized by containing at least one of astaxanthin and tocotrienols as an effective ingredient, too.

As the forms of foods and drinks, there may be taken an example of adding the effective ingredient to general foods such as margarine, butter, butter sauce, cheese, raw cream, shortening, lard, ice cream, yogurt, diary products, meat sauce products, fish products, pickles, fried potato, potato chips, snack confectionery, sliced and dried rice cake, popcorn, a seasoned powder for sprinkling over rice, chewing gum, chocolate, pudding, jelly, gumi-candy, candy, drops, caramel, bread, sponge cake, cake, doughnut, biscuit, cookie, cracker, etc., macaroni, pasta, Chinese noodles, buck wheat, wheat vermicelli, salad oils, instant soup, dressing, egg, mayonnaise, miso, etc., or carbonated or non-carbonated drinks such as fruit drinks, refreshing drinks, sports drinks, etc., non-alcoholic drinks such as tea, coffee, cocoa, etc., or liquors such as liqueur, medical liquor, etc.

The food and drink of the present invention can be prepared by incorporating at least one of astaxanthin and tocotrienols in the raw materials for the general food and processing the mixture according to the conventional method. The amount incorporated of astaxanthin and tocotrienols is not particularly restricted and it may be varied depending on the form of food and so on. In general, the total amount of astaxanthin and 0.00001-10 % by weight, preferably 0.00001-5 % by weight and it is adjusted so as to contain only the amount necessary to exert the preventive or alleviative effect. The total amount to be used of at least one of astaxanthin and tocotrienols can be selected appropriately depending on the kinds of food and drink by a person having ordinary skill in the art, and it is 0.5-400 mg, preferably 1-40 mg per day for an adult.

When the food and drink of the present invention are used as a nutritional supplement food or a healthy food, their forms may be the same as the form of the above-described medicament. There may also be used milk protein, soybean protein, egg albumin protein, etc., or their decomposed material such as albumen oligopeptide, soybean hydrolyzate, mixture of amino acid unit. The food can also be formed into natural liquid foods, semi-digested nutritional foods and nutritional foods, drinks, capsules, enteral nutrients, etc. by combining with sugars, fats, trace elements, vitamins, emulsifying agents, flavors, etc. For the drink form to be provided, there can be incorporated nutritional additives such as amino acids, vitamins, minerals, etc., and sweetening agents, spices, flavors, pigments, etc., in order to keep a balance in the nutrients or to impart good taste when taking.

The present invention includes an animal feed having an effect of alleviating, treating and preventing vascular failure and an effect of protecting vascular endothelial cells which are characterized by containing at least one of astaxanthin and tocotrienols as an effective ingredient, too

The feed of the present invention is not particularly restricted to a solid preparation, solid, pellet shaped, granular, biscuit shaped or paste form, a dry food, a semidry food (e.g. feed containing about 10-50 % by weight of water), a wet food (e.g. feed containing about 50-80 % by weight of water), etc. It may be prepared by adding at least one of astaxanthin and tocotrienols to a material for feed and mixing together or by sprinkling an aqueous solution of at least one of astaxanthin and tocotrienols on a feed at an appropriate step of the processes hitherto produced the feed. The feed of the present invention may be prepared by adding at least one of astaxanthin and tocotrienols to a commercial feed and mixing together or by sprinkling an aqueous solution of at least one of astaxanthin and tocotrienols on the commercial feed. Also, it may be prepared in the form of easily eatable solid preparation such as tablet, sublingual tablet, pill, triturate, powder, fine granule, granule, capsule, soft capsule, etc. like the nutritional supplement food for a human.

As the raw material to be incorporated, it is not restricted particularly if it can be used as a raw material for a feed. As the raw material for a feed, at least one kind of the ingredients which are conventionally used may be used for incorporation depending on the kind of feed. Examples of such ingredients include animal raw materials such as fish flour, fish meat, fish and fish shellfish, fish mea, livestock meal, meat meal, meat and bone meal, blood meal, feather meal, silk worm chrysalis oil meal, dried skimmilk, animal fat (cattle oil, pig oil, bone oil and the like), chicken eggs, milks, etc.; microorganisms such as brewer's yeast, Tolula yeast and the like; gains such as corn, milo, wheat, barley, rye, oat, wheat flour, brown rice, millet, soybean, toasted soybean flour, cassava and the like; starches such as pregelatinized starch, starch and the like; oil meals such as soybean meal, dehulled soybean meal, rapeseed meal, peanut meal, coconut meal, sunflower meal, linseed meal, sesama meal, safflower meal, palm nuclear meal, kapok seed meal and the like; brans such as rice bran, barley bran, wheat bran and the like; by-product feeds such as gluten feed, gluten meal, starch pulp, purified honey, soy sauce cake, brewers grain, beet pulp, bagasse, tofu byproduct, malt sprouts, orange peel, orange juice cake and the like; fibers such as alfalfa meal, timothy hay, straw and the like; an excipient, a binding agent, a disintegrant, a salt, saccharides such as sugar and the like, vitamins, amino acids, minerals, etc.

As raw materials which may be incorporated into a solid preparation, in addition to the before-mentioned raw materials, for example a carrier, which is generally used in the field of food for a human, may be mixed uniformly therein for production. Specifically, saccharides such as sucrose, sorbitol, fructose and the like; polyethylene glycol, propylene glycol and the like; oils such as sesame oil, rapeseed oil, olive oil, soybean oil and the like; flavors such as strawberry flavor, peppermint and the like may be used for production. It may be prepared in the form of triturate, pill, capsule, soft capsule or tablet using excipients such as lactose, glucose, sucrose, lactose, manitol, corn starch, silicon dioxide and the like; disintegrants such as starch, sodium alginate and the like; lubricants such as magnesium stearate, talc and the like; binding agents such as polyvinyl alcohol, hydroxypropyl cellulose, gelatin, casein and the like; emulsifying agents such as glycerin fatty acid ester, sucrose fatty acid eater, sorbitan fatty acid ester, saponin, lecithin and the like; thickening agents such as guar gum, alginic acid, agar, pectine, gum arabic, crystalline cellulose and the like; plasticizers such as glycerin and the like.

The feed of the present invention may contains additives such as a fortifier, a quality-improving agent, an antibiotic, a bactericide, an enzyme, a fungicide, an antioxidant, a coloring agent, a sweetener, a perfume and so on.

Although the content of astaxanthin and tocotrienols in the feed of the present invention may be varied by the form of feed and is not restricted particularly, it can be selected within the range not damaging the palatability. In general, the total content of astaxanthin and tocotrienols is 0.00001-10 % by weight, preferably 0.00001-5 % by weight.

The amount fed of the feed to an animal can be selected depending on age, body weight and so on. For example, the total amount of astaxanthin and tocotrienols is about 1-500 µg/day, preferably about 2-300 µg/day, more preferably about 5-200 µg/day based on 1 kg of body weight. Incidentally, the feed is fed to an animal at any time and it may be fed in one portion per day or it may be fed in plural portions per day.

The present invention may be applied to a variety of animals. Examples of an animal include *Manmalia* such as a mouse, a rat, a guinea pig, a hamster, a rabbit, a monkey, a dog, a cat, a pig, a cattle, a sheep, a horse and the like; *Reptilia* such as a crocodile, a snake, a lizard and the like; birds such as a chicken, a parakeet, a parrot, a mina bird, a pigeon and the like; Amphilia such as a salmon, trout, a tuna, a porgy, a guppy and the like.

### Best Mode for Carrying out the Invention

The present invention will be hereinafter described in greater detail with reference to Examples, but it is needless to say that it is not restricted to these Examples only.

### Example 1

### [Preparation of a culture medium]

9.4 g a minimum essential medium (EMEM) of Eagle powder (a product of Nihon Pharmaceutical Co., Ltd.) was dissolved in 1000 ml of water at room temperature under stirring and sterilized in an autoclave (121 °C for 15 minutes). A sodium bicarbonate solution (a product of Otsuka Pharmaceutical Meylon Inc.), glutamine (a product of Nissui Pharmaceutical Co., Ltd.), an essential amino acid solution (a product of Gibco Company), a non-essential amino acid solution (a product of Gibco Company), a mixed vitamin solution (a product of Gibco Company) and a mixed antibiotic solution (a product of Gibco Company) were added thereto. To this culture medium was added a cattle's fatal serum (a product of Gibco Company) to prepare 10 % and 0.4 % content of cattle's fatal serum (hereinafter, referred to as "10 % FBS added EMEM", "0.4 % FBS added EMEM", respectively).

### [Test method: 24-wells plate]

Vascular endothelial cells (GM07373A) subcultured with 10 % FBS added EMEM were separated with a trypsin-EDTA solution. To 1.5 ml of the solution of the cells floated on 10 % FBS added EMEM was added 9 ml of 10 % FBS added EMEM and the resultant mixture was placed in a laboratory dish having a diameter of 9 cm and pre-cultivation was conducted at 37 °C for 3-4 days under an atmosphere of 5 % carbon dioxide. Vascular endothelial cells in the culture broth were separated with a trypsin-EDTA solution and floated on 10 % FBS added EMEM. 1 ml of the floated cell solution (number of cell: 0.7-0.9 x 105 cell/ml) was poured into each well of 24-wells plate and the main cultivation was conducted at 37 °C for 2 days under an atmosphere of 5 % carbon dioxide. After removal of the culture medium, 0.4 % FBS added EMEM was added in an amount of 500 µl per well to make the cells in starvated state and cultivation was conducted at 37 °C over a night under an atmosphere of 5 % carbon dioxide. A sample solution and 10 µl of an aqueous glucose solution (200 mg/ml) were added and cultivation was conducted at 37 °C for 2 hours under an atmosphere of 5 % carbon dioxide. After removal of the culture medium, A Hanks' solution (a product of Nihon Pharmaceutical Co., Ltd.) was added to the wells in the amount of 1 ml per well for twice washing. After removal of the solution, a dye solution containing hydrogen peroxide (or a dye solution not containing hydrogen peroxide) was added in the amount 300 µl per well and cultivation was conducted at 37 °C for 30 minutes under an atmosphere of 5 % carbon dioxide. The dye solution was removed to measure the fluorescent intensity of the cell.

GM07373A is a cattle's aortic vascular endothelial cell strain, "Repository No. GM07373A" purchased from Coriel Human Genetic Cell Repository in U.S.A. The dye solution containing hydrogen peroxide was prepared by mixing 50 µg of the solution of fluorescent pigment ("C-6827", a product of Molecular Probe Company) dissolved in 90 µl of DMSO with 9 ml of the Hanks' solution and adding 90 µl of 0.3 % hydrogen peroxide thereto. Hydrogen peroxide was not added for control test.

As sample solution, there were used methanol solutions of astaxanthin (a product of Sigma Company) in 0.053 mg/ml and 0.0053 mg/ml, methanol solutions of α-tocopherol (a product of Wako Pure Chemical Industries, Ltd.) in 0.25 mg/ml and 0.025 mg/ml and methanol solutions of tocotrienol (a product of Sigma Company) in 0.25 mg/ml and 0.025 mg/ml. 10 µl of each of the sample solution was added to 500 µl of the culture medium.

### [Method for measuring fluorescent intensity: graphic analysis software method]

The fluorescent intensity was measured at the optional three places under 100 magnifications using a Leica stereoscopic fluorescence microscope "MZ FL III" (having Leica macro fluorescence apparatus having "GFP" attachment) and a filter set (name): GEP Plant fluorescence (GFP2) (excitation filter: 480/40 nm, absorption filter: 510nm). Each of the pictures was captured by a CDD camera and converted into gray intensity % to measure fluorescent intensity. Fluorescent intensity was measured for three places per well and the average value was calculated. Also, 3-4 wells were prepared for a kind of sample.

**[Table 1] Fluorescent intensity when astaxanthin was added**

| Sample added (amount added) | Fluorescent intensity (%) |
|---|---|
| None | 1.59 |
| Hydrogen peroxide | 2.22 |
| Glucose | 1.25 |
| Hydrogen peroxide + Glucose | 4.51 |
| Astaxanthin (0.104 µg/ml) | 2.55 |
| Astaxanthin (1.04 µg/ml) | 1.93 |

The system where astaxanthin has added is one where astaxanthin in addition to hydrogen peroxide plus glucose was added. The period of time for pre-cultivation was 5 days and number of cell was 0.84 x 10⁵ cell per well.

The fluorescent intensity increased by the addition of hydrogen peroxide plus glucose. This indicates that the generated active oxygen dyed fluorescent upon oxidation of fluorescent pigment and the vascular endothelial cells were injured. Contrary thereto, the system where astaxanthin had been further added to the hydrogen peroxide plus glucose added system has less fluorescent intensity than the hydrogen peroxide plus glucose added system. This indicates that astaxanthin has an effect for suppressing injury of the vascular endothelial cells by the generated active oxygen.

**[Table 2] Fluorescent intensity when tocotorienol and α-tocopherol were added**

| Specimen added (amount added) | Fluorescent intensity (%) |
|---|---|
| None | 0.21 |
| Hydrogen peroxide | 8.69 |
| Glucose | 0.26 |
| Hydrogen peroxide + Glucose | 9.00 |
| Tocotorienol (0.5 µg/ml) | 4.35 |
| Tocotorienol (5.0 µg/ml) | 4.40 |
| α-Tocopherol (0.5 µg/ml) | 4.74 |
| α-Tocopherol (5.0 µg/ml) | 4.32 |

The tocotorienol or α-tocopherol added system is one where tocotorienol or α-tocopherol in addition to hydrogen peroxide plus glucose was added. The period of time for pre-cultivation was 3 days and number of cell was 0.89 x 10⁵ cell per well.

The addition of hydrogen peroxide plus glucose brings about the increase in the fluorescent intensity. This indicates that the generated active oxygen dyed fluorescent and the vascular endothelial cells were injured. Contrary thereto, the system where tocotorienol or α-tocopherol has been further added to the hydrogen peroxide plus glucose added system has less fluorescent intensity than the hydrogen peroxide plus glucose added system. This indicates that tocotorienol or α-tocopherol has an effect for suppressing the injury of the vascular endothelial cells by the generated active oxygen.

### Example 2

### [Test method: 96-wells plate]

Although the method is basically the same as that in Example 1, 96-wells plate was used and the staged dilution was conducted with a multipippte, and a fluorescent plate reader was employed. That is, vascular endothelial cells subculcured in a T25 flask were separated with a trypsin-EDTA solution and diluted with 10 % FBS added EMEM culture medium. And then, pre-cultivation was conducted for 4 days in a T25 flask. The cells in confluent state were again separated with a trypsin-EDTA solution and diluted with 10 % FBS added EMEM culture medium to prepare a cell floated solution of 95 x 104 cell/ml. The cell floated solution was sprinkled over the 96-wells plate in the amount of 100 µl per well and cultivation was conducted for 2 days. The culture medium was removed under suction and 50 µl of 0.4 % FBS added EMEM culture medium was added to the residue and thereafter the cells in starvated state was cultivated over a night. The culture medium was removed under suction and using 0.4 % FBS added EMEM culture medium containing glucose (the final concentration: 400 mg/dl) the dilution rank (50 µl/well, 2 times x 8 stages) for each sample was prepared by a multipippete, and each of the sample solutions was added to its designated wells. The sample solution was prepared by dissolving adequately each of samples in methanol according to similar manner as in Example 1 to prepare an original sample solution. The original sample solution was diluted with 0.4 % FBS added EMEM for use. After addition of a sample solution, a stimulation cultivation was conducted at 37 °C for 2 hours in a 5 % carbon dioxide cultivation tank. The culture medium was removed under suction and washing was conducted twice with 100 µl of a Hanks' solution per well. And thereafter, a solution prepared by adding to a Hanks' solution a fluorescent pigment (C-6827) dispersed in DMSO and hydrogen peroxide solution (the final concentration:0.003 %) according to the similar manner as in Example 1 was added to the 96-wells plate in the amount of 30 µl per well. The cultivation was conducted for 25 minutes in a 5 % carbon dioxide cultivation tank to introduce the fluorochrome into the cells. The pigment solution was removed under suction and the residue was allowed to stand over a night under an interruption of light. The fluorescent intensity was measured with Fluostar plate reader. Incidentally, the diluted rank of hydrogen peroxide (the final concentration: 100 µM) was added for use as the standard.

### [Method for measuring fluorescent intensity: a direct measurement method]

FluoStar ("BMC", a product of Labtech Company) was used for measuring fluorescent intensity. The measurement of fluorescent intensity was conducted using FluoStar ("BMC", a product of Labtech Company) which was an apparatus for measuring automatically fluorescent intensity at every well. The fluorescent intensity was measured under the condition of excitation of 485 nm and emission of 538 nm using xenon lamp as a light source. The fluorescent intensity when light of 485 nm was flashed 10 times was represented as the electric pressure and the well containing 100 µM of hydrogen peroxide having a strongest fluorescent intensity was adjusted to 8,000 (no unit) as the gain.

**[Table 3] Fluorescent intensity (24-wells plate) when tocotorienol and astaxanthin were added**

| Astaxanthin | Tocotorienol [µg/ml] | | |
|---|---|---|---|
| [µg/ml] | 10 | 100 | 1000 |
| 0.053 | 5619 | 5231 | 5129 |
| 0.159 | 5589 | 4896 | 4887 |

The fluorescent intensity when hydrogen peroxide and glucose were added is 7655 while the fluorescent intensity when hydrogen peroxide and glucose were not added was 1338. The fluorescent intensity was measured by a direct measurement method using 24-wells plate.

It can be seen from the results shown in table 3 that as the amount of astaxanthin is greater, the value of fluorescent intensity becomes less. When the amount added of astaxanthin is smaller in comparison with that of tocotorienol, the value of fluorescent intensity becomes less. This indicates that by administering astaxanthin and tocotorienol together, synergistic effect of protecting vascular cells is achieved.

**[Table 4] Fluorescent intensity (24-wells plate) tocopherol and astaxanthin were added**

| Astaxanthin | Tocopherol [µg/ml] | | |
|---|---|---|---|
| [µg/ml] | 10 | 100 | 1000 |
| 0.053 | 6799 | 6555 | 6238 |
| 0.159 | 5809 | 5778 | 5700 |

The fluorescent intensity when hydrogen peroxide and glucose were added is 7605 while the fluorescent intensity when hydrogen peroxide and glucose were not added was 1210. The fluorescent intensity was measured by a direct measurement method using 24-wells plate.

It can be seen from the results shown in table 4 that as the amount of astaxanthin and tocopherol is greater, the value of fluorescent intensity becomes less. When the amount added of astaxanthin is smaller in comparison with that of tocopherol, the value of fluorescent intensity becomes less. This indicates that by administering astaxanthin and tocopherol together, synergistic effect of protecting vascular cells is achieved.

**[Table 5] Fluorescent intensity (96-wells plate) when glutathion and astaxanthin were added**

| Astaxanthin | Glutathion [µg/ml] | | |
|---|---|---|---|
| [µg/ml] | 370.3 | 1111.0 | 3333.0 |
| 19.7 | 6394 | 6311 | 6292 |
| 177.0 | 6248 | 5856 | 5668 |

The fluorescent intensity when hydrogen peroxide and glucose were added is 5250 while the fluorescent intensity when hydrogen peroxide and glucose were not added was 6689. The fluorescent intensity was measured by a direct measurement method using 96-wells plate.

It can be seen from the results shown in table 4 that as the amount of astaxanthin and glutathion is greater, the value of fluorescent intensity becomes less. When the amount added of astaxanthin is smaller in comparison with that of glutathion, the value of fluorescent intensity becomes less. This indicates that by administering astaxanthin and glutathion together, synergistic effect of protecting vascular cells is achieved.

### [Preparation Example 1] Tablet

The following ingredients were uniformly mixed together in the following composition ratio (% by weight) to make tablets, each 200 mg of weight.

| | |
|---|---|
| Astareal powder | 10 parts by weight |
| Powdered blueberry | 2 parts by weight |
| V premix | 3 parts by weight |
| Lactose | 50 parts by weight |
| Potato Starch | 32 parts by weight |
| Polyvinyl alcohol | 2 parts by weight |
| Magnesium stearate | 1 parts by weight |

| | |
|---|---|
| Astareal powder (a product of Fuji Chemical Industrial Co., Ltd.) is the powder product prepared from Hematococcus alga extract oil containing 1 % by weight of astaxanthin in terms of free form. | |

### [Preparation Example 2] Capsule

Hematococcus alga extract oil (astaxanthin content of 5 % by weight) was filled in the outer shell of soft capsule consisting of the following ingredients according to the conventional method to make soft capsules, each 200 mg of weight.

| | |
|---|---|
| Gelatin | 70 parts by weight |
| Glycerin | 23 parts by weight |
| Propyl paraoxy benzoate | 0.5 part by weight |
| Water | 6.5 parts by weight |

### [Preparation Example 3] Drink

The following ingredients were compounded together according to the conventional method to prepare a drink.

| | |
|---|---|
| Hematococcus alga extract oil | 5 parts by weight |
| Tocotrienol oil | 5 parts by weight |
| Sodium tartarate | 0.1 part by weight |
| Liquid sugar | 800 parts by weight |
| Citric acid | 12 parts by weight |
| Vitamin C | 10 parts by weight |
| Vitamin E | 30 parts by weight |
| Perfume | 1.5 part by weight |
| Potassium chloride | 0.1 part by weight |
| Magnesium sulfate | 0.5 part by weight |
| Water | 10000 parts by weight |

Hematococcus alga extract oil contains 5 % by weight of astaxanthin in terms of free astaxanthin, and tocotrienol oil contains 40 % by weight of tocotrienol.

### [Preparation Example 4] Cookie

The following ingredients were compounded and baked according to the conventional method to prepare cookies.

| | |
|---|---|
| Astareal powder | 10 parts by weight |
| Cow's milk | 630 parts by weight |
| Sugar | 130 parts by weight |
| Corn starch | 130 parts by weight |
| Salt | 10 parts by weight |

### [Preparation Example 5] Feed

The following ingredients were compounded and formed into pellets to make a feed for kitchen.

| | |
|---|---|
| Astareal powder | 10 parts by weight |
| Corn powder | 300 parts by weight |
| Wheat flour | 300 parts by weight |
| Fish meal | 50 parts by weight |
| Alfalfa meal | 50 parts by weight |
| Cassava meal | 50 parts by weight |
| Wheat bran | 50 parts by weight |
| Soybean powder | 200 parts by weight |

### Brief Description of the Drawings

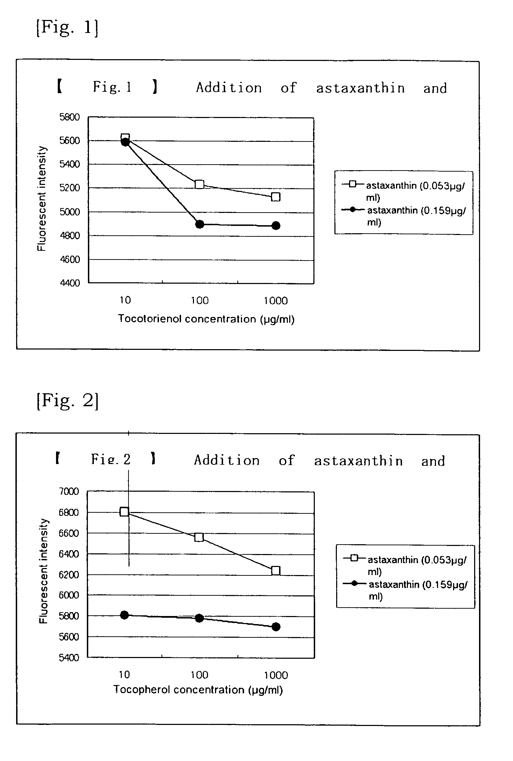

[Fig. 1] is a graph showing fluorescent intensity when tocotorienol and astaxanthin in table 3 were added. Tocotorienol concentration is expressed in an index.
[Fig. 2] is a graph showing fluorescent intensity when tocotorienol and astaxanthin in table 4 were added. Tocotorienol concentration is expressed in an index.

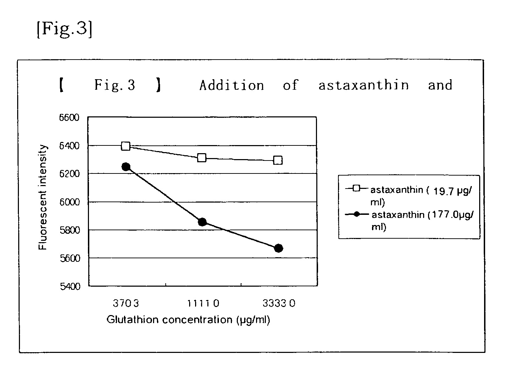

[Fig. 3] is a graph showing fluorescent intensity when glutathion and astaxanthin in table 5 were added.

## Claims

1. An agent for alleviating vascular failure which is **characterizing by** containing at least one of astaxanthin and tocotrienols as an effective ingredient.

2. A vascular endothelial cell-protecting agent which is **characterizing by** containing at least one of astaxanthin and tocotrienols as an effective ingredient.

3. A drink and a food each having an effect of alleviating vascular failure which is **characterizing by** each containing at least one of astaxanthin and tocotrienols as an effective ingredient.

4. A drink and a food each having an effect of protecting a vascular endothelial cell which is **characterizing by** each containing at least one of astaxanthin and tocotrienols as an effective ingredient.

5. An animal feed having an effect of alleviating vascular failure which is **characterizing by** containing at least one of astaxanthin and tocotrienols as an effective ingredient.

6. An animal feed having an effect of protecting a vascular endothelial cell which is **characterizing by** containing at least one of astaxanthin and tocotrienols as an effective ingredient.
